# FASCICULE DE BREVET EUROPEEN

(11) **EP 1 377 322 B1**
(45) Date de publication et mention de la délivrance du brevet: **17.02.2010**
(21) Numéro de dépôt: 02722365.0
(22) Date de dépôt: 22.03.2002
(51) Int. Cl.: A61L 2/00, A23L 3/04

(54) **UNITE DE STERILISATION**
VORRICHTUNG ZUM STERILISIEREN
STERILISING UNIT

(30) Priorité: 09.04.2001 FR 0104875
(43) Date de publication de la demande: 07.01.2004
(73) Titulaire: Camu, Patrice, 67700 Saverne (FR)
(72) Inventeur: Camu, Patrice, 67700 Saverne (FR)
(74) Mandataire: Rhein, Alain
(86) Numéro de dépôt international: PCT/FR2002/001014
(87) Numéro de publication internationale: WO 2002/080988

(56) Documents cités:
- FR-A- 2 172 816
- US-A- 3 606 995
- US-A- 5 619 908

## Description

La présente invention a trait à une unité de stérilisation pour produits alimentaires, pharmaceutiques ou autres, conditionnés dans des contenants rigides ou souples, comprenant au moins un autoclave dans lequel est prévu apte à accueillir, au travers d'au moins une porte d'accès et à l'aide d'une unité de chargement-déchargement, au moins un conteneur dans lequel ont été disposés lesdits contenants.

L'invention concerne le domaine de la stérilisation de substances conditionnées dans des contenants rigides, tels des bouteilles, flacons, bocaux, ou souples, tels des pochettes souples soudées thermiquement, dans le cadre d'une chaîne de production de produits alimentaires, pharmaceutiques, vétérinaires ou autres contenus dans de tels emballages.

De telles unités de stérilisation sont connues dans le cadre des sites de production agroalimentaires ou pharmaceutiques. Un certain nombre d'autoclaves sont regroupés en une ou plusieurs unités de stérilisation en aval du processus alimentaire ou pharmaceutique, c'est à dire une fois que le produit se trouve dans son conditionnement commercial, avant la pose de l'étiquette et la mise en cartons ou sous films plastiques dans le cas d'une boîte de conserve traditionnelle par exemple.

Lesdits autoclaves sont généralement de forme cylindrique allongée et disposés à l'horizontale. Afin d'optimiser le fonctionnement du site industriel considéré, les autoclaves sont disposés parallèlement côte à côte, de façon à ce que leurs portes d'accès, habituellement situées à une de leurs extrémités, soient alignées, perpendiculairement à un circuit de chargement-déchargement organisé en boucle.

Chaque autoclave est alimenté, en début de cycle, en conteneurs, par exemple des palettes, remplies de contenants de substances à stériliser. Ladite alimentation est assurée par une unité de chargement et par l'intermédiaire d'un moyen de transport approprié pouvant être, entre autres, un tapis, un rail, un convoyeur à chaînes, ou une ligne filoguidée. Une organisation plus vétuste mettra en oeuvre des chariots porte-palettes.

De façon analogue, chaque autoclave est déchargé en fin de cycle par une unité de déchargement, pouvant être éventuellement identique à ladite unité de chargement, par l'intermédiaire d'un moyen de transport d'évacuation. A l'intérieur de chaque autoclave, un dispositif adéquat permet la circulation et le positionnement des différentes palettes, comme cela se fait dans les avions cargos, à l'aide de rouleaux cylindriques par exemple.

L'ensemble de ces opérations nécessite une synchronisation fine entre les cycles de fonctionnement des différents autoclaves, les temps de chargement et déchargement, ainsi que les temps de préparation et de libération des conteneurs. Ainsi, ceux-ci doivent, avant stérilisation, être chargés en produits provenant des halls de fabrication, par exemple des bocaux ou des sachets, et, après stérilisation, être déchargés desdits produits stérilisés, en partance pour l'étiquetage, l'encartonnage ou le filmage, puis le stockage avant livraison.

Précisons que le cycle de stérilisation comprend des actions thermiques et mécaniques. Les actions thermiques consistent à faire subir aux produits, disposés dans leur conteneur, par ailleurs calés sur des supports appropriés, des cycles de montée et descente en température, lesdits cycles étant induits par des moyens tels que l'envoi de vapeur dans l'enceinte de l'autoclave, et inversement l'envoi d'eau froide venant doucher les produits. Les actions mécaniques consistent, en phase de stérilisation, à générer, des vibrations, des oscillations ou des retournements des produites.

Le circuit en boucle du local de stérilisation permet ou non les dépassements de conteneurs afin d'optimiser les temps d'attente devant chaque autoclave. Cette organisation n'est pas sens présenter un certain nombre d'inconvénients, dont certains sont directement liés aux autoclaves :
- Premièrement, du fait de leur disposition horizontale, les autoclaves occupent une surface conséquente au sol, à la fois pour eux-mêmes et pour la zone d'évolution attenante.

Le document US 3 606 995 décrit en premier lieu un tel autoclave dont l'axe longitudinal est orienté horizontalement et apporte une solution aux inconvénient d'une telle disposition au travers d'un autoclave dont l'enceinte est disposée verticalement.
- Deuxièmement, le chargement et le déchargement desdits conteneurs à partir d'une porte située en extrémité d'autoclave présuppose un moyen de déplacement horizontal idoine efficace, généralement au sol, ainsi qu'une optimisation de la hauteur, de la largeur et de la longueur des différents chargements en fonction des dimension utiles des autoclaves. Ce moyen peut être relativement complexe et encombrant.
- Troisièmement, l'enveloppe des autoclaves chargés doit être prévue apte à supporter, outre les cycles de température et de pression, les contraintes générées par le poids de chargement.

A cet effet, l'invention concerne une unité de stérilisation pour produits alimentaires, pharmaceutiques ou autres, conditionnés dans des contenants rigides ou souples, comprenant au moins un autoclave prévu apte à accueillir, au travers d'au moins une porte d'accès et à l'aide d'une unité de chargement-déchargement, au moins un conteneur dans lequel on été disposés lesdits contenants.

Selon l'invention, l'autoclave est disposé verticalement et comporte, intérieurement, des moyens de transports verticaux susceptibles d'être soumis, à l'aide de moyens moteurs adaptés, à une progression pas à pas pour leur chargement et leur déchargement, au travers de la ou des portes d'accès, de conteneurs sous forme de clayettes prévues aptes à être chargées et déchargées en contenants rigides ou souples par ladite unité de chargement-déchargement.

Selon une autre caractéristique de l'invention, lesdits moyens de transport verticaux sont constitués d'au moins trois vis sans fin, dont au moins deux sont situées de part et d'autre de l'espace de déplacement des conteneurs par rapport à un plan passant par l'axe vertical dudit autoclave et sensiblement par l'axe médian de la ou les portes d'accès, et dont au moins une s'étend verticalement du côté de cet espace de déplacement des conteneurs qui est opposé à celui en regard de la ou desdites portes.

Selon une autre caractéristique de l'invention, ledit ou lesdits conteneurs sont munis de rebords d'appui prévus aptes à coopérer avec le filetage des vis sans fin.

Selon une autre caractéristique de l'invention, lesdits conteneurs sont guidés verticalement par l'intermédiaire de rails de guidage s'étendant parallèlement à la ou aux vis sans fin.

Selon une autre caractéristique de l'invention, les deux vis sans fin situées de part et d'autre de l'espace de déplacement des conteneurs, ont des pas de vis inversés de manière apte à être soumises à une rotation en sens contraire, déterminée pour pousser les conteneurs en contact avec la troisième vis sans fin.

Selon une autre caractéristique de l'invention, lesdits moyens de transports verticaux sont prévus aptes à supporter intégralement le poids des conteneurs de produits à stériliser et à le transmettre au sol par des embases.

Selon une autre caractéristique de l'invention, ladite unité de stérilisation est munie de moyens de mise en oscillation des conteneurs.

Avantageusement, les moyens de mise en oscillation desdits conteneurs sont constitués par au moins une des vis sans fin, laquelle est soumise à une rotation alternée d'amplitude déterminée.

L'invention sera mieux comprise à la description qui va suivre et au vu du dessin ci-joint se rapportant à un mode de réalisation.
La figure 1 est une représentation schématisée et en coupe verticale d'un autoclave d'une unité de stérilisation selon l'invention.
La figure 2 est une représentation schématisée et en coupe selon II-II de la figure 1 ;
La figure 3 est une représentation schématisée et en élévation des moyens de chargement-déchargement des autoclaves de l'unité de stérilisation.

Tel que représenté dans les figures du dessin ci-joint, la présente invention concerne le domaine des unités de stérilisation pour produits alimentaires, pharmaceutiques ou autres, conditionnés dans des contenants rigides ou souples, comprenant au moins un autoclave 1.

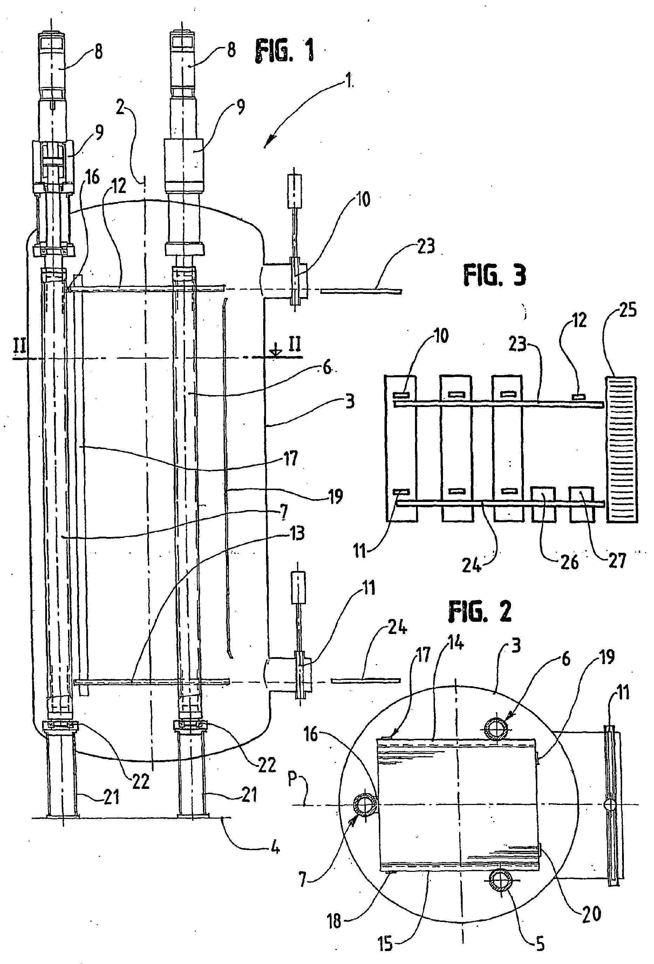

Celui-ci, représenté en coupe verticale sur la figure 1, et en coupe transversale perpendiculaire sur la figure 2, est du type cylindrique, l'axe longitudinal 2 de son enceinte 3 étant vertical. Des moyens de transport verticaux, plus particulièrement des vis sans fin 5,6,7, visibles dans la figure 2, sont disposés verticalement au sein de cette enceinte 3.

Sur ces vis sans fin 5, 6, 7 agissent des moyens moteurs permettant de les mettre en rotation dans un sens ou dans un autre, lesdits moyens moteurs comprenant par exemple un moteur électrique 8, associé à un réducteur 9.

Préférentiellement, deux portes horizontalement oblongues 10, 11 sont agencées dans un même alignement vertical, latéralement dans la paroi délimitant l'enceinte 3 de l'autoclave 1. Ces portes sont dimensionnées de façon à autoriser le passage de conteneurs 12 lors des manutentions, tant au chargement qu'au déchargement. Un tel conteneur 12, en l'occurrence une clayette, a été représenté en coupe, dans la figure 1, dans la partie supérieure de l'autoclave 1 en regard d'une porte supérieure. De façon analogue, un autre conteneur 13, en l'occurrence également une clayette, est représenté en coupe dans la partie inférieure de l'autoclave en regard d'une porte inférieure 11, ladite porte 11 étant également visualisée sur la figure 2.

Au moins deux des vis sans fin 5,6 sont agencées de part et d'autre de l'espace de déplacement des clayettes 12, 13 par rapport à un plan P vertical, passant par l'axe vertical dudit autoclave 1 et sensiblement par l'axe médian de sa ou ses portes 10, 11, ledit plan vertical P correspondant au plan de coupe de la figure 1. Par ailleurs au moins une troisième vis 7 est, elle, préférentiellement, située dans ledit plan P, du côté, par rapport à cet espace de déplacement des clayettes 12, 13, opposé à celui desdites portes 10,11.

Afin d'assurer leur positionnement, les clayettes ou conteneurs 12, 13 sont munies, en périphérie, de rebords d'appui 14, 15, 16 prévus aptes à coopérer avec les filetages des vis sans fin 5, 6, 7.

Dans le but d'assurer un guidage et un positionnement optimal des clayettes 12,13 lors des opérations de manutention, il peut être avantageux d'encadrer la butée représentée par la vis sans fin 7 par deux rails guides latéraux verticaux 17, 18 séparés d'une distance équivalant à la largeur d'une clayette 12, rebords d'appui 14,15 compris, augmentée du jeu prévu.

De façon analogue et afin d'assurer un guidage et un positionnement optimal des clayettes 12, 13 lors des opérations de positionnement vertical à l'intérieur de l'enceinte 3, il peut être avantageux de disposer au moins un rail guide vertical 19, 20 situé, par rapport à l'espace de déplacement de ces clayettes 12, 13, du côté des portes d'accès 10, 11. Ce ou ces rails guides verticaux 19, 20 sont destinés, entre autres, à conserver le rebord d'appui 16 de la clayette 12, 13 en appui sur le filetage de la vis sans fin 7. Bien entendu, lesdits rails guides verticaux 19,20 s'étendent, limitativement, dans la section de l'enceinte 3 libre de toute ouverture de portes, afin de ne pas venir gêner ou empêcher le passage des conteneurs 12, 13.

Les deux vis sans fin 5,6 situées de part et d'autre de l'espace de déplacement des clayettes, tel que décrit antérieurement, ont avantageusement des pas de vis inversés et il leur est communiqué une rotation en sens contraire ayant pour effet de pousser les clayettes 12, 13 en contact avec la troisième vis sans fin 7 située tel que décrit antérieurement.

Lesdites vis sans fin 5, 6, 7 peuvent être montées, par l'intermédiaire de roulements 22 admettant tout particulièrement les efforts axiaux, sur des embases 21 émergeant sous l'autoclave 1 pour reposer au sol. Aussi, ces vis sans fin 5, 6, 7 supportent intégralement le poids des conteneurs de produits à stériliser.

Les moyens de chargement-déchargement évoqués précédemment comprennent au moins des convoyeurs 23, 24 s'étendant horizontalement au droit des portes d'accès 10, 11 des autoclaves 1 composant ladite unité de stérilisation. Lesdits moyens sont intégrés dans un circuit plus complexe de manutention, schématisé en figure 3.

Si, selon un exemple de réalisation, les clayettes 12,13 de produits à stériliser sont chargées par la porte supérieure 10 et si le déchargement s'effectue par la porte inférieure 11, les opérations de manutention et de transport sont les suivantes: les clayettes 12, 13 de produits à stériliser sont chargées sur un poste de chargement 27, puis gagnent, par l'intermédiaire d'un ascenseur 25, un convoyeur supérieur 23, avant d'être introduites au travers de ladite porte supérieure 10 à l'intérieur de autoclave 1 en cycle de chargement. Le cycle de stérilisation terminé, lesdites clayettes 12,13 sont extraites de cet autoclave 1 au travers d'une porte inférieure 11 et gagnent un poste de déchargement 26 en empruntant un convoyeur inférieur 24.

Ainsi, les clayettes 12 sont introduites une par une par la porte supérieure 10 à partir du convoyeur supérieur 23. Une rotation idoine des vis 5, 6, 7 les fait baisser de l'équivalent de la hauteur d'une clayette chargée, augmentée d'un intervalle de sécurité, ce qui est finement obtenu par un contrôle de rotation pas à pas. L'opération se poursuit jusqu'au remplissage de l'enceinte 3. Pour vider celle-ci, on peut, soit inverser l'opération, soit la poursuivre en utilisant la porte inférieure 11. Evidemment, les opérations précédentes peuvent être inversées en travaillant à partir de la porte inférieure 11 et en utilisant, soit ladite porte inférieure 11, soit la porte supérieure 10 en déchargement.

Le fait d'avoir une ou plusieurs portes intermédiaires permet d'avoir plusieurs points de chargement simultanés, chaque rotation des vis permettant d'abaisser ou d'élever identiquement une ou plusieurs piles de clayettes 12 au sein de l'autoclave 1, ce qui permet de diminuer considérablement les temps de manutention sur des autoclaves de grande hauteur.

Ledit autoclave 1 peut autoriser la mise en oscillation des clayettes 12, 13, ce type de mouvement permettant d'améliorer les effets thermiques du cycle de stérilisation. Par exemple, au moins une des vis sans fin 5, 6, 7, peut être mise en rotation alternée dans un sens, puis en sens inverse, selon une amplitude déterminée. Cela a pour effet d'incliner alternativement les clayettes dans un sens puis dans l'autre. Il convient de respecter une inclinaison maximale compatible avec la géométrie des pièces mécaniques en contact.

Afin de refroidir les clayettes 12 lorsque requis au cours d'un cycle de stérilisation, l'enceinte 3 peut être munie de rampes d'arrosage verticales, ici non représentées.

## Revendications

1. Unité de stérilisation pour produits alimentaires ou pharmaceutiques, conditionnés dans des contenants rigides ou souples, comprenant au moins un autoclave (1) disposé verticalement et dans lequel prend position, au travers d'au moins une porte d'accès (10,11), au moins un conteneur (12,13) sous forme de clayette dans lequel ont été disposés lesdits contenants au travers d'une unité de chargement - déchargement (23), ledit autoclave (1) comportant intérieurement des moyens de transports verticaux soumis, à l'aide de moyens moteurs adaptés (8,9), à une progression pas à pas pour le chargement et le déchargement dudit conteneur (12,13) au travers de ladite porte d'accès (10,11), **caractérisé par le fait que** lesdits moyens de transport verticaux sont constitués d'au moins trois vis sans fin disposée verticalement.

2. Unité de stérilisation selon la revendication 1, **caractérisé par le fait qu'**au moins deux vis (5,6) sont situées de part et d'autre de l'espace de déplacement du conteneur (12,13) par rapport à un plan (P) passant par l'axe vertical dudit autoclave (1) et sensiblement par l'axe médian ou les portes (10,11), et dont au moins une vis (7) s'étend verticalement du côté de cet espace de déplacement du conteneur (12,13) qui est opposé à celui de la ou desdites portes (10,11) .

3. Unité de stérilisation selon l'une quelconque des revendications 1 ou 2, **caractérisé par le fait que** ledit conteneur (12,13) est muni de rebords d'appui (14,15,16) prévus aptes à coopérer avec le filetage des vis sans fin (5,6,7).

4. Unité de stérilisation selon l'une quelconque des revendications précédentes, **caractérisé par le fait que** ledit conteneur (12,13) est guidé verticalement par l'intermédiaire de rails de guidage (12,13).

5. Unité de stérilisation selon la revendication 2, **caractérisé par le fait que** les deux vis sans fin (5,6) situées de part et d'autre de l'espace de déplacement du conteneur (11,12) ont des pas de vis inversés de manière à être soumises à une rotation en sens contraire, déterminée pour pousser le conteneur (12,13) en contact avec la vis sans fin (7).

6. Unité de stérilisation selon l'une quelconque des revendications précédentes, **caractérisé par le fait que** lesdits moyens de transports verticaux (5,6,7) sont prévus aptes à supporter intégralement le poids de plusieurs conteneurs (12,13) et à le transmettre au sol par des embases.

7. Unité de stérilisation selon l'une quelconque des revendications précédentes, **caractérisé par le fait qu'**il est muni d'une ou plusieurs portes latérales (10,11) et de moyens de chargement-déchargement (23,24,25) pour acheminer, d'une part, les clayettes (12,13) à stériliser vers un autoclave (1) depuis une unité de chargement (27) et, d'autre part, pour évacuer lesdites clayettes (12,13) après stérilisation vers une unité de déchargement (26) depuis cet autoclave (1).

8. Unité de stérilisation selon l'une quelconque des revendications précédentes, **caractérisé par le fait que** ledit autoclave (1) est muni de moyens de mise en oscillation des conteneurs (12,13).

9. Unité stérilisation selon la revendication 8, **caractérisé par le fait que** les moyens de mise en oscillation desdits conteneurs (12,13) sont constitués par au moins une des vis sans fin (7,8 ou 9), laquelle est soumise à une rotation alternée d'amplitude déterminée.

10. Unité de stérilisation selon l'une quelconque des revendications précédentes, **caractérisé par le fait qu'**elle est munie de rampes d'arrosage verticales.

## Claims

1. Sterilising unit for food or pharmaceutical products packaged in rigid or flexible containers, comprising at least one vertically arranged autoclave (1) in which is positioned, through at least one access door (10, 11), at least one container (12, 13) in the form of a tray in which said containers have been arranged through a loading - unloading unit (23), said autoclave (1) including internally vertical transport means subjected, through adapted driving means (8, 9), to a stepwise advancing for loading and unloading said container (12, 13) through said access door (10, 11), wherein said vertical transport means are formed by at least three worms arranged vertically.

2. Sterilising unit according to claim 1, wherein at least two worms (5, 6) are located on both sides of the space of displacement of the container (12, 13) with respect to a plane (P) passing through the vertical axis of said autoclave (1) and substantially through the median axis or the doors (10, 11), and at least one worm (7) of which extends vertically on the side of this space of displacement of the container (12, 13) opposite to that of said door or doors (10, 11).

3. Sterilising unit according to any of claims 1 or 2, wherein said container (12, 13) is provided with resting rims (14, 15, 16) designed capable of cooperating with the thread of the worms (5, 6, 7).

4. Sterilising unit according to any of the preceding claims, wherein said container (12, 13) is guided vertically through guiding rails (12, 13).

5. Sterilising unit according to claim 2, wherein the two worms (5, 6) located on both sides of the space of displacement of the container (11, 12) have reversed pitches so as to be subjected to a rotation in opposite direction, determined so as to push the container (12, 13) into contact with the worm (7).

6. Sterilising unit according to any of the preceding claims, wherein said vertical transport means (5, 6, 7) are designed capable of fully supporting the weight of several containers (12, 13) and of transmitting same to the floor through bases.

7. Sterilising unit according to any of the preceding claims, wherein it is provided with one or several side doors (10, 11) and loading - unloading means (23, 24, 25) for conveying, on the one hand, the trays (12, 13) to be sterilised from a loading unit (27) towards an autoclave (1) and, on the other hand, for evacuating said trays (12, 13) after sterilisation from this autoclave (1) towards an unloading unit (26).

8. Sterilising unit according to any of the preceding claims, wherein said autoclave (1) is provided with means for putting the containers (12, 13) into oscillation.

9. Sterilising unit according to claim 8, wherein the means for putting said containers (12, 13) into oscillation are formed by at least one of the worms (7, 8 or 9), which is subjected to an alternating rotation of a determined amplitude.

10. Sterilising unit according to any of the preceding claims, wherein it is provided with vertical spraying ramps.

## Patentansprüche

1. Vorrichtung zum Sterilisieren von in starren oder biegsamen Behältern verpackten Nahrungsmitteln oder pharmazeutischen Produkten, umfassend wenigstens einen senkrecht angeordneten Autoklav (1), in dem durch wenigstens eine Zugangstür (10, 11) hindurch wenigstens ein Behälter (12, 13) in der Form eines Kastenträgers angeordnet ist, in dem die besagten Behälter über eine Belade-/Entladeeinheit (23) angeordnet wurden, wobei der besagte Autoklav (1) inwendig senkrechte Fördermittel umfaßt, die über geeignete Treibmittel (8, 9) einem stufenweise Fortschritt ausgesetzt werden, um den besagten Behälter (12, 13) durch die besagte Zugangstür (10, 11) hindurch zu laden und zu entladen, **dadurch gekennzeichnet, dass** die besagten senkrechte Fördermittel durch wenigstens drei Schnecken gebildet sind, die senkrecht angeordnet sind.

2. Vorrichtung zum Sterilisieren nach Anspruch 1, **dadurch gekennzeichnet, dass** wenigstens zwei Schnecken (5, 6) sich beiderseits des Verschiebungsraums des Behälters (12, 13) bezüglich einer Ebene (P) befinden, die durch die senkrechte Achse des besagten Autoklavs (1) und im wesentlichen durch die Mittelachse oder die Türen (10, 11) läuft, und von denen sich wenigstens eine Schnecke (7) senkrecht an der Seite dieses Verschiebungsraums des Behälters (12, 13) erstreckt, die derjenigen der besagten Tür bzw. Türen (10, 11) gegenüberliegt.

3. Vorrichtung zum Sterilisieren nach irgendeinem der Ansprüche 1 oder 2, **dadurch gekennzeichnet, dass** der besagte Behälter (12, 13) mit hochstehenden Auflagerändern (14, 15, 16) versehen ist, die geeignet vorgesehen sind, um mit dem Gewinde der Schnecken (5, 6, 7) zusammenzuwirken.

4. Vorrichtung zum Sterilisieren nach irgendeinem der vorgehenden Ansprüche, **dadurch gekennzeichnet, dass** der besagte Behälter (12, 13) über Führungsschienen (12, 13) senkrecht geführt wird.

5. Vorrichtung zum Sterilisieren nach Anspruch 2, **dadurch gekennzeichnet, dass** die sich beiderseits des Verschiebungsraums des Behälters (11, 12) befindlichen zwei Schnecken (5, 6) umgekehrte Gewindesteigungen aufweisen, sodass sie einer Drehung in Gegenrichtung ausgesetzt werden, die bestimmt wird, um den Behälter (12, 13) in Berührung mit der Schnecke (7) zu drängen.

6. Vorrichtung zum Sterilisieren nach irgendeinem der vorgehenden Ansprüche, **dadurch gekennzeichnet, dass** die besagten senkrechten Fördermittel (5, 6, 7) geeignet vorgesehen sind, um das Gewicht von mehreren Behältern (12, 13) völlig zu tragen und dieses über Basisplatten dem Boden zu übertragen.

7. Vorrichtung zum Sterilisieren nach irgendeinem der vorgehenden Ansprüche, **dadurch gekennzeichnet, dass** sie mit einer oder mehreren Seitentüren (10, 11) und mit Belade-/Entlademitteln (23, 24, 25) versehen ist, um einerseits die zu sterilisierenden Kastenträger (12, 13) von einer Beladeeinheit (27) zu einem Autoklav (1) zu fördern und andererseits die besagten Kastenträger (12, 13) nach der Sterilisierung von diesem Autoklav (1) zu einer Entladeeinheit (26) abzuführen.

8. Vorrichtung zum Sterilisieren nach irgendeinem der vorgehenden Ansprüche, **dadurch gekennzeichnet, dass** der besagte Autoklav (1) mit Mitteln versehen ist, um die Behälter (12, 13) in Oszillation zu bringen.

9. Vorrichtung zum Sterilisieren nach Anspruch 8, **dadurch gekennzeichnet, dass** die Mittel, um die besagten Behälter (12, 13) in Oszillation zu bringen, durch wenigstens eine der Schnecken (7, 8 oder 9) gebildet sind, die einer Wechseldrehung einer bestimmten Amplitude ausgesetzt ist.

10. Vorrichtung zum Sterilisieren nach irgendeinem der vorgehenden Ansprüche, **dadurch gekennzeichnet, dass** sie mit senkrechten Sprührampen versehen ist.
